(19) Europäisches Patentamt
European Patent Office
Office européen des brevets

(11) Numéro de publication: **0 370 901 B1**

# (12) FASCICULE DE BREVET EUROPEEN

(45) Date de publication de fascicule du brevet: **01.02.95**

(51) Int. Cl.[6]: **C07D 311/22**, C07D 311/68, C07D 311/70, C07F 9/655, A61K 31/35

(21) Numéro de dépôt: **89403220.0**

(22) Date de dépôt: **22.11.89**

(54) **Nouveaux dérivés du chromanne actifs sur le système nerveux central, leur procédé de préparation et les compositions pharmaceutiques en contenant.**

(30) Priorité: **23.11.88 FR 8815284**

(43) Date de publication de la demande: **30.05.90 Bulletin 90/22**

(45) Mention de la délivrance du brevet: **01.02.95 Bulletin 95/05**

(84) Etats contractants désignés: **AT BE CH DE FR GB IT LI LU NL SE**

(56) Documents cités: **EP-A- 0 093 535**

(73) Titulaire: **SANOFI**
**32-34, rue Marbeuf**
**F-75008 Paris (FR)**

(72) Inventeur: **Garcia, Georges**
**27, rue des Aires**
**F-34980 Saint-Gely-du-Fesc (FR)**
Inventeur: **Di Malta, Alain**
**170, rue de Vigne Belle**
**St-Clément-La-Rivière**
**F-34980 Saint-Gely-du-Fesc (FR)**
Inventeur: **Soubrie, Philippe**
**Le Rey**
**Valflaunès**
**F-34270 Saint Mathieu de Treviers (FR)**

(74) Mandataire: **Gillard, Marie-Louise et al**
**Cabinet Beau de Loménie**
**158, rue de l'Université**
**F-75340 Paris Cédex 07 (FR)**

**EP 0 370 901 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des dérivés du chromanne et du chromène présentant une activité sur le système nerveux central, notamment en tant qu'antidépresseurs.

Le brevet belge 829 611 mentionne toute une série de dérivés du chromannol-3 caractérisés par la présence en position 4 d'un groupe $-NR_1R_2$, dans lequel $R_1$ est l'hydrogène ou un groupe hydrocarboné éventuellement substitué, $R_2$ représente l'hydrogène ou un groupe alkyle ou encore le groupe $-NR_1R_2$ représente un groupe hétérocyclique comportant de 3 à 8 atomes et éventuellement substitué par un ou deux groupes méthyle.

Ces dérivés comportent en outre éventuellement une grande variété de substituants en position 6 et en position 7.

La demande de brevet européen publiée sous le numéro 76 075 décrit des dérivés du chromannol-3 caractérisés par la présence en position 4 d'un groupe oxo-2 pyrrolidinyl-1 ou d'un groupe oxo-2 pipéridino et par la présence éventuelle de substituants très variés en position 6 ou en position 7.

La demande de brevet européen 93 535 décrit des dérivés du chromène caractérisés par la présence en position 4 d'un groupe oxo-2 pipéridino ou oxo-2 pyrrolidinyl-1 et par la présence éventuelle d'une grande variété de substituants en position 6 ou en position 7.

La demande de brevet européen 273 262 décrit une série de dérivés du chromannol-3 et de dérivés du chromène caractérisés par la présence en position 4 d'un groupe oxo-2 hétérocyclique choisi parmi la pyridone, la pyrazinone, la pyridazinone, la pyrimidinone et la thiopyridone ainsi que parmi les cycles correspondants partiellement hydrogénés, tous ces cycles pouvant être éventuellement substitués.

Ces dérivés de chromannol et de chromène sont en outre éventuellement substitués en position 6 ou 7.

La demande de brevet européen 296 975 décrit des dérivés du chromannol-3 également caractérisés par la présence en position 4 d'un groupe oxo-2 hétérocyclique. Enfin, la demande de brevet européen 312 432 décrit des dérivés du chromène substitués en position 4 par un groupe oxo-2 pyridyl-1.

Tous les composés décrits dans ces brevets ou demandes de brevets présentent en commun une activité importante sur le système cardiovasculaire et spécialement en tant qu'antihypertenseurs.

On a maintenant trouvé selon la présente invention qu'en modifiant la nature du substituant en position 4 on obtenait de façon surprenante des composés n'ayant qu'une activité faible ou nulle sur le système cardiovasculaire, mais possédant une activité très intéressante sur le système nerveux central et notamment en tant qu'antidépresseurs.

Les composés selon l'invention sont les composés répondant à la formule générale :

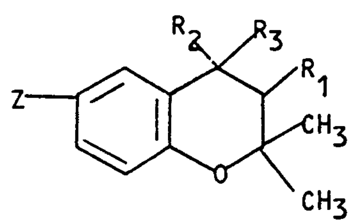

(I)

dans laquelle :

- Z représente un halogène ou un groupe cyano, acétyle, trifluoroacétyle, nitro, alkylthio en $C_1$-$C_4$, carboxy, phosphono, dialcoxyphosphonyle, alcoxycarbonyle, le groupe alcoxy contenant de 1 à 3 atomes de carbone;
- $R_1$ désigne un groupe hydroxyle et $R_2$ représente l'hydrogène, ou encore $R_1$ et $R_2$ pris ensemble constituent une liaison supplémentaire entre les atomes de carbone qui les portent;
- $R_3$ représente
  . un groupe $NH$-$X$-$R_4$ dans lequel X désigne une liaison directe ou un groupe $SO_2$ et $R_4$ représente un groupe phényle non substitué ou substitué 1 ou 2 fois par un halogène, un groupe nitro, un groupe alkyle en $C_1$-$C_4$, un groupe trifluorométhyle, un groupe amino, un groupe $-N(Alk)_2$, un groupe -COOAlk, un groupe OAlk, dans lesquels Alk désigne un groupe alkyle en $C_1$-$C_4$;
  . un groupe $-S-R_5$ où $R_5$ représente un groupe alkyle en $C_1$-$C_4$, un groupe cycloalkyle en $C_3$-$C_7$, un groupe phényle non substitué ou substitué 1 ou 2 fois par un halogène, un groupe nitro, un groupe alkyle en $C_1$-$C_4$, un groupe trifluorométhyle, un groupe amino, un groupe $-N(Alk)_2$, un groupe -COOAlk, un groupe OAlk, dans lesquels Alk désigne un groupe alkyle en $C_1$-$C_4$; ou encore $R_5$ désigne un groupe $-AlkN(Alk)_2$ où Alk est tel que défini ci-dessus;

. un groupe $SO_2R_5$ où $R_5$ est tel que défini ci-dessus;
ainsi que les sels que les composés de formule (I) sont susceptibles de fournir avec les acides pharmaceutiquement acceptables lorsque le substituant $R_3$ comprend un groupe amino.

Dans la présente description et dans les revendications qui vont suivre, on entend par halogène un atome de fluor, de chlore ou de brome.

La présente invention a également pour objet un procédé de préparation des composés de formule (I).

Lorsque $R_1$ représente un hydroxyle, les chromannols-3 dans lesquels $R_3$ est un groupe $NHR_4$ ou $SR_5$ sont obtenus en traitant l'époxyde

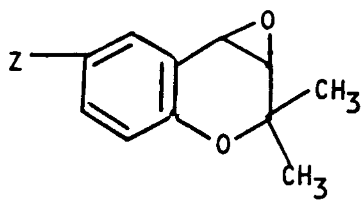

(II)

par l'amine $R_4NH_2$ ou le thiol correspondant $HSR_5$.

La réaction d'ouverture de l'époxyde (II) est conduite à une température comprise entre 10 et 100 °C dans un solvant organique inerte, comme un alcool, le dioxanne, le tétrahydrofuranne, le méthyl-tertiobutyléther, le diméthylsulfoxyde ou le diméthylformamide en présence d'un agent de condensation basique, tel que l'hydrure de sodium ou un hydroxyde d'ammonium quaternaire, comme l'hydroxyde de benzyltriméthylammonium.

Dans ces conditions opératoires, l'ouverture de l'époxyde (II) conduit à un dérivé chromannol-3 de configuration trans.

Les époxydes de départ (II) sont connus; ils sont décrits notamment dans le brevet belge n° 852 955 et dans la demande de brevet européen 296 975.

Lorsque $R_3$ représente un groupe $-NHSO_2R_4$, il n'est pas possible d'ouvrir l'époxyde par le sulfonamide correspondant. Dans ce cas, les composés (I) correspondants sont obtenus à partir de dérivés de l'amino-4 diméthyl-2,2 hydroxy-3 chromanne (III) par substitution du groupe amino par le sulfochlorure $R_4SO_2Cl$ selon le schéma :

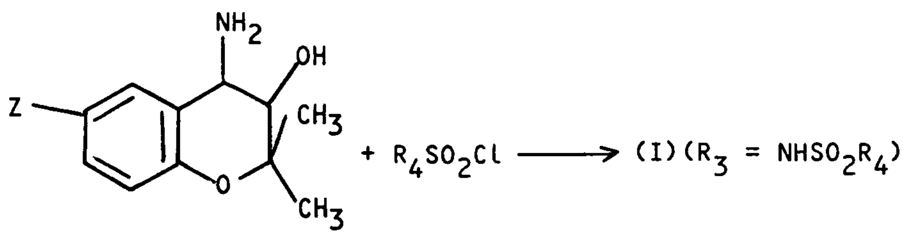


(III)

La réaction est effectuée au sein d'un solvant, capable de fixer l'acide chlorhydrique qui se forme, tel que la pyridine, à une température comprise entre 20 et 50 °C.

Les dérivés aminés (III) sont connus ou préparés selon la méthode décrite en particulier dans la demande de brevet EP 76 075, par ouverture de l'époxyde (II) par l'ammoniac.

Enfin, lorsque $R_3$ représente un groupe $SO_2R_5$, les composés (I) correspondants sont obtenus par oxydation par l'eau oxygénée ou un peracide organique des composés (I), où $R_3$ est un groupe $-SR_5$, préparés comme indiqué ci-dessus. On opère de façon habituelle dans un solvant inerte, tel que le chloroforme à une température peu élevée (10 à 30 °C).

Lorsque $R_1$ et $R_2$ représentent ensemble une liaison, les dérivés de chromène de formule (I) sont obtenus par déshydratation des chromannols-3 correspondants.

La déshydratation est effectuée par un hydrure alcalin tel que l'hydrure de sodium dans un solvant inerte, tel que le tétrahydrofuranne à une température comprise entre 50 et 100 °C.

Dans le cas où Z = CN, selon une variante du procédé, on peut partir d'un bromo-3 cyano-6 hydroxy-4 chromanne qu'on traite par une amine $NH_2R_4$, un thiol $HSR_5$ (ou son sel alcalin) en présence d'un agent de

condensation, tel que l'hydrure de sodium, au sein d'un solvant comme le diméthylsulfoxyde.

On effectue ainsi en une seule étape :

- la formation in situ de l'époxyde (II) à partir de la bromhydrine de départ,
- l'ouverture de l'époxyde par l'amine ou le thiol,
- la déshydratation du chromannol-3 obtenu ci-dessus en chromène.

Les exemples suivants non limitatifs illustrent la préparation des composés de formule (I).

EXEMPLE 1

Trans (chloro-4 anilino)-4 cyano-6 diméthyl-2,2 hydroxy-3 chromanne (SR 44546).

(I) : Z = CN, $R_1$ = OH, $R_2$ = H,

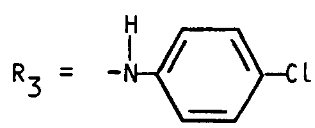

On chauffe à reflux pendant 72 heures la solution de 3 g de cyano-6 diméthyl-2,2 époxy-3,4 chromanne et 2,1 g de parachloroaniline dans 50 ml d'éthanol.

On évapore le solvant sous vide et reprend le résidu dans l'éther. On lave la solution avec de l'eau, sèche et évapore le solvant. Le résidu cristallise dans l'hexane. On recristallise dans l'éther isopropylique.

Poids : 1 g, F. : 125 °C.

EXEMPLE 2

Trans cyano-6 diméthyl-2,2 hydroxy-3 (méthoxycarbonyl-2 anilino)-4 chromanne (SR 44588).

(I) : Z = CN, $R_1$ = OH, $R_2$ = H,

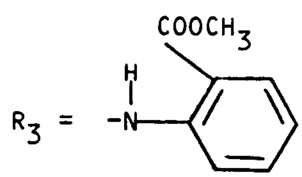

On opère comme dans l'exemple 1 en remplaçant la parachloroaniline par une quantité équivalente d'anthranilate de méthyle et en limitant la période de chauffage à 18 heures. De la même façon, on obtient le produit attendu.

F. : 164 °C.

EXEMPLE 3

Trans cyano-6 diméthyl-2,2 hydroxy-3 (trifluorométhyl-2 benzènesulfonamido-1)-4 chromanne (SR 44665).

(I) : Z = CN, $R_1$ = OH, $R_2$ = H,

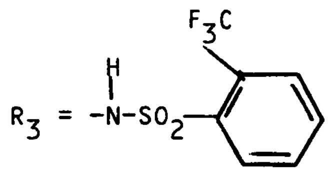

On agite à température ambiante pendant 18 h le mélange de 1 g d'amino-4 cyano-6 diméthyl-2,2 hydroxy-3 chromanne et 1 g de trifluorométhyl-2 benzènesulfochlorure dans 25 ml de pyridine.

On évapore la pyridine et reprend le résidu dans le chlorure de méthylène. On lave la solution organique avec une solution diluée d'acide chlorhydrique, puis sèche sur sulfate de sodium et élimine le solvant sous vide.

Le résidu solide est recristallisé dans un mélange éther isopropylique-isopropanol.

Poids : 0,9 g, F. : 211-212 °C.

EXEMPLE 4

Transcyano-6 diméthyl-2,2 (N,N-diméthylsulfamoylamino)-4 hydroxy-3 chromanne (SR 44666).

(I) : Z = CN, $R_1$ = OH, $R_2$ = H,

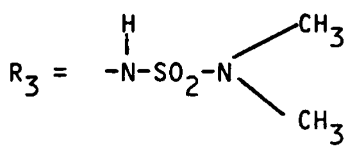

On opère comme dans l'exemple 3 en remplaçant le trifluorométhyl-2 benzènesulfochlorure par une quantité équivalente de chlorure de N,N-diméthylsulfamoyle.

F. : 172-173 °C (éther isopropylique).

EXEMPLE 5

Trans (chloro-4 phénylthio)-4 cyano-6 diméthyl-2,2 hydroxy-3 chromanne (SR 44611).

(I) : Z = CN, $R_1$ = OH, $R_2$ = H,

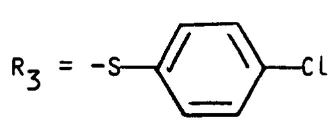

On chauffe au reflux pendant 5 heures le mélange de 2 g de cyano-6 diméthyl-2,2 époxy-3,4 chromanne et 1,8 g de parachlorothiophénol dans 15 ml de dioxanne et 0,3 ml d'une solution méthanolique à 35% d'hydroxyde de benzyltriméthylammonium.

On évapore le solvant sous vide et reprend le résidu dans le pentane. Après cristallisation, on recristallise dans l'éther isopropylique.

F. : 143 °C.

EXEMPLE 6

Trans cyano-6 diméthyl-2,2 (diméthylamino-2 éthylthio)-4 hydroxy-3 chromanne, chlorhydrate (SR 44652 A)

(I) : Z = CN, $R_1$ = OH, $R_2$ = H,

$R_3$ = 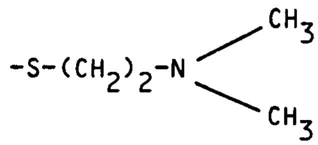

On chauffe au reflux, pendant 2 heures, le mélange de 2 g de cyano-6 diméthyl-2,2 époxy-3,4 chromanne et 3 g de chlorhydrate de diméthylamino-2 éthanethiol dans 15 ml d'éthanol et 12 ml d'une solution méthanolique à 35% d'hydroxyde de benzyltriméthylammonium.

On évapore les solvants sous vide et reprend le résidu dans 30 ml d'eau, puis extrait avec de l'éther. On sèche la solution éthérée sur sulfate de sodium et évapore le solvant sous vide.

On obtient une huile (4 g) qu'on dissout dans 8 ml d'éthanol. On ajoute 1,45 ml d'acide chlorhydrique concentré, puis 60 ml d'éther.

On obtient un solide incolore (4 g).

F. : 169 ° C. Cristallisé avec 0,5 molécule d'eau.

EXEMPLE 7

(Chloro-4 phénylthio)-4 cyano-6 diméthyl-2,2 2H-chromène (SR 44554).

(I) : Z = CN, $R_1$ + $R_2$ = liaison,

$R_3$ = 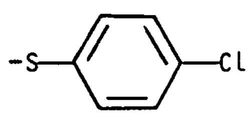

On agite à 15 ° C le mélange de 7 g de bromo-3 cyano-6 diméthyl-2,2 hydroxy-4 chromanne et 30 ml de diméthylsulfoxyde. On ajoute 0,6 g d'hydrure de sodium et poursuit l'agitation à 15 ° C pendant 1 heure.

On ajoute 4,6 g de parachlorothiophénate de sodium et agite le mélange pendant 20 heures à 20 ° C. On verse le mélange réactionnel sur 150 g de glace et extrait avec de l'éther. On lave la solution organique avec une solution de soude 1 N, puis avec une solution d'acide chlorhydrique 1 N.

On sèche la solution sur sulfate de sodium et évapore le solvant sous vide. Le résidu huileux cristallise. On le recristallise une première fois dans l'éther isopropylique et une seconde fois dans l'isopropanol.

On obtient des cristaux incolores.

F. : 117 ° C.

EXEMPLE 8

Trans cyano-6 cyclohexylsulfonyl-4 diméthyl-2,2 hydroxy-3 chromanne (SR 44708).

(I) : Z = CN, $R_1$ = OH, $R_2$ = H,

$R_3$ = 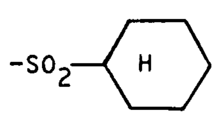

A) Trans cyano-6 cyclohexylthio-4 diméthyl-2,2 hydroxy-3 chromanne.

On chauffe au reflux, pendant 8 heures, le mélange de 1,5 g de cyano-6 diméthyl-2,2 époxy-3,4 chromanne, 1,2 g de cyclohexylmercaptan et 0,3 g d'une solution méthanolique à 35% d'hydroxyde de benzyltriméthylammonium dans 10 ml de tétrahydrofuranne.

On évapore à siccité sous vide et reprend le résidu dans 50 ml d'éther. On lave la solution avec de l'eau, sèche sur sulfate de sodium et évapore le solvant. Il reste une huile qu'on reprend dans 50 ml de pentane bouillant. Par refroidissement à 0 ° C, on obtient des cristaux incolores.

F. : 101 ° C.

B) SR 44708

On maintient pendant 2 heures à 20 ° C le mélange de 1,2 g du produit préparé ci-dessus et 4,8 g d'acide métachloroperbenzoïque dans 50 ml de chloroforme.

On ajoute 100 ml de chlorure de méthylène et lave la solution organique avec une solution à 10% de carbonate de sodium, puis avec de l'eau. On sèche la solution et évapore les solvants sous vide. On obtient un solide qu'on recristallise dans l'éthanol absolu.

F. : 202 ° C.

EXEMPLES 9 A 23

En suivant le mode opératoire de l'exemple 1, on a préparé les composés selon l'invention décrits dans le tableau I ci-dessous :

TABLEAU I

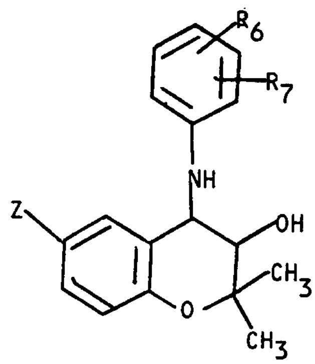

| Composé n° SR (Exemple) | Z | $R_6$ | $R_7$ | Point de fusion (solvant) |
|---|---|---|---|---|
| SR 46063 (9) | $NO_2$ | H | Cl-4 | F. : 183°C ($(iPr)_2O$) |
| SR 46142 A (10) | CN | H | H | chlorhydrate F. : 185°C (acétonitrile) |
| SR 46143 (11) | CN | H | F-4 | F. : 125°C ($(iPr)_2O$) |
| SR 46144 (12) | CN | H | Cl-3 | Fc : 155°C ($(iPr)_2O$) |
| SR 46145 (13) | CN | H | $CH_3$-4 | F. : 111°C ($(iPr)_2O$) |
| SR 46146 (14) | CN | H | $OCH_3$-4 | F. : 173°C (AcOEt) |
| SR 46276 (15) | CN | H | $CF_3$-4 | RMN |
| SR 46344 (16) | $NO_2$ | H | Cl-3 | F. : 170°C |
| SR 46357 (17) | CN | H | F-3 | F. : 128°C ($(iPr)_2O$) |
| SR 46358 (18) | CN | H | Br-3 | hémihydrate F. : 160-163°C |
| SR 46462 (19) | CN | Cl-2 | Cl-4 | F. : 168°C ($(iPr)_2O$-hexane |

TABLEAU I (suite)

| Composé n° SR (Exemple) | Z | $R_6$ | $R_7$ | Point de fusion (solvant) |
|---|---|---|---|---|
| SR 46463 (20) | CN | Cl-3 | $CH_3$-4 | F. : 163 °C ($(iPr)_2O$-hexane) |
| SR 46482 (21) | CN | H | $NO_2$-3 | F. : 163-165 °C ($(iPr)_2O$-hexane) |
| SR 46483 (22) | CN | H | $NH_2$-3 | F. : 173 °C ($(iPr)_2O$-hexane) |
| SR 46568 (23) | CN | H | $N(CH_3)_2$-3 | F. : 77-79 °C (hexane) |

Pour les solvants de recristallisation, on a utilisé les abréviations suivantes :

$(iPr)_2O$ : éther isopropylique

AcOEt : acétate d'éthyle

Le composé SR 46276 a été caractérisé par son spectre de RMN.

Les produits selon l'invention ont été étudiés en ce qui concerne leur activité thérapeutique.

Ainsi, les produits selon l'invention ont été soumis au test de la nage forcée selon Porsolt et al. (Archives Internationales de Pharmacodynamie 1977, 229, 327, 336).

Des souris femelles (Iffa Credo) de 22-24 g sont plongées pour une durée de 6 minutes dans un bécher contenant 800 ml d'eau à 24 ± 1 °C.

Les temps d'immobilité sont mesurés pendant 4 minutes de la 2$^e$ à la 6$^e$ minute.

Les résultats sont exprimés en pourcentage d'inhibition des temps d'immobilité des animaux traités vis-à-vis des animaux témoins.

Dans le tableau II ci-dessous sont indiqués les résultats obtenus avec divers produits selon l'invention.

TABLEAU II

| Produit | Doses mg/kg voie i.p. | % d'inhibition |
|---|---|---|
| SR 44546 | 0,06<br>0,25 | 16<br>44 |
| SR 44588 | 0,06<br>0,125 | 20<br>27 |
| SR 44665 | 0,06 | 24<br>34 |
| SR 44666 | 0,06<br>1 | 21<br>23 |
| SR 44652 | 0,06<br>0,125 | 25<br>27 |
| SR 44554 | 0,06 | 33 |
| SR 44708 | 0,06<br>0,25 | 21<br>37 |
| SR 46142 A<br>SR 46143<br>SR 46276 | 0,06<br>0,06<br>0,06 | 40<br>27<br>40 |

Ces résultats montrent que les produits testés sont doués de propriétés antidépressives se manifestant à des doses faibles.

Par ailleurs, les produits selon l'invention ont été étudiés en ce qui concerne leurs propriétés cardiovasculaires et notamment leurs propriétés antihypertensives. Chez le rat spontanément hypertendu, les produits n'ont montré aucune activité, même à des doses très supérieures à celles où ils sont actifs sur le système nerveux central.

Enfin, les produits selon l'invention ne manifestent aucun signe de toxicité aux doses où ils sont actifs.

Les produits selon l'invention sont donc utilisables en médecine en tant que médicaments actifs sur le système nerveux central.

Selon un second aspect, la présente invention concerne des compositions pharmaceutiques contenant une dose efficace d'un composé de formule (I) avec des excipients convenables.

Les excipients utilisés sont choisis selon la forme pharmaceutique et le mode d'administration souhaité.

Dans les compositions pharmaceutiques de la présente invention pour l'administration orale, sublinguale, sous-cutanée, intramusculaire, intraveineuse ou rectale, les principes actifs de formule (I) ci-dessus, ou leurs sels éventuels, peuvent être administrés sous formes unitaires d'administration, en mélange avec des supports pharmaceutiques classiques, aux êtres humains pour la prophylaxie ou le traitement des troubles ou des maladies ci-dessus. Les formes unitaires d'administration appropriées comprennent les formes par voie orale telles que les comprimés, les gélules, les poudres, les granulés et les solutions ou suspensions orales, les formes d'administration buccale, les formes d'administration sous-cutanée, intramusculaire ou intraveineuse et les formes d'administration rectale.

Afin d'obtenir l'effet prophylactique ou thérapeutique désiré, la dose de principe actif peut varier entre 0,5 et 25 mg par kg de poids du corps et par jour.

Chaque dose unitaire peut contenir de 5 à 500 mg, de préférence de 25 à 300 mg, d'ingrédients actifs en combinaison avec un support pharmaceutique. Cette dose unitaire peut être administrée 1 à 5 fois par jour de façon à administrer un dosage journalier de 25 à 2 500 mg, de préférence de 125 à 1 500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Le principe actif peut être formulé également sous forme de microcapsules, éventuellement avec un ou plusieurs supports ou additifs.

A titre d'exemple de préparation galénique, on peut préparer des gélules contenant :

| | |
|---|---|
| - SR 44546 | 0,050 g |
| - Lactose | 0,100 g |
| - Stéarate de magnésium | 0,025 g |

en mélangeant intimement les ingrédients ci-dessus et en versant le mélange dans des gélules de gélatine dure.

## Revendications

**1.** Composés de formule :

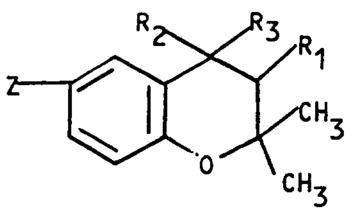

(I)

dans laquelle :

- Z représente un halogène ou un groupe cyano, acétyle, trifluoroacétyle, nitro, alkylthio en $C_1$-$C_4$, carboxy, phosphono, dialcoxyphosphonyle, alcoxycarbonyle, les groupes alkylthio et alcoxy contenant de 1 à 3 atomes de carbone;
- $R_1$ désigne un groupe hydroxyle et $R_2$ représente l'hydrogène, ou encore $R_1$ et $R_2$ pris ensemble constituent une liaison supplémentaire entre les atomes de carbone qui les portent;
- $R_3$ représente
  . un groupe NH-X-$R_4$ dans lequel X désigne une liaison directe ou un groupe $SO_2$ et $R_4$ représente un groupe phényle non substitué ou substitué 1 ou 2 fois par un halogène, un groupe nitro, un groupe alkyle en $C_1$-$C_4$, un groupe trifluorométhyle, un groupe amino, un groupe -N(Alk)$_2$, un groupe -COOAlk, un groupe OAlk, dans lesquels Alk désigne un groupe alkyle en $C_1$-$C_4$;
  . un groupe -S-$R_5$ où $R_5$ représente un groupe alkyle en $C_1$-$C_4$, un groupe cycloalkyle en $C_3$-$C_7$, un groupe phényle non substitué ou substitué 1 ou 2 fois par un halogène, un groupe nitro, un groupe alkyle en $C_1$-$C_4$, un groupe trifluorométhyle, un groupe amino, un groupe -N(Alk)$_2$, un groupe -COOAlk, un groupe OAlk, dans lesquels Alk désigne un groupe alkyle en $C_1$-$C_4$; ou encore $R_5$ désigne un groupe -AlkN(Alk)$_2$ où Alk est tel que défini ci-dessus;
  . un groupe $SO_2R_5$ où $R_5$ est tel que défini ci-dessus;

  ainsi que les sels que les composés de formule (I) sont susceptibles de fournir avec les acides pharmaceutiquement acceptables lorsque le substituant $R_3$ comprend un groupe amino.

**2.** Le trans (chloro-4 anilino)-4 cyano-6 diméthyl-2,2 hydroxy-3 chromanne.

**3.** Le (chloro-4 phénylthio)-4 cyano-6 diméthyl-2,2 2H-chromène.

**4.** Procédé de préparation des composés de formule (I) dans laquelle $R_3$ représente un groupe NH-$R_4$, $SR_5$ ou $SO_2R_5$, caractérisé en ce que :

- on fait réagir sur l'époxyde

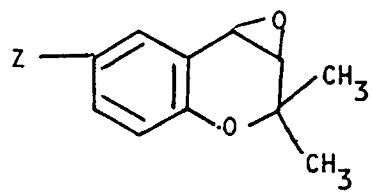

(II)

une amine $R_4NH_2$ ou un thiol $HSR_5$ au sein d'un solvant inerte à une température comprise entre 10 et 100 °C et en présence d'un agent basique tel que l'hydrure de sodium ou l'hydroxyde de benzyltriméthylammonium pour former le chromannol (I) où $R_3$ représente un groupe NH-$R_4$ ou $SR_5$;
- éventuellement on oxyde par l'eau oxygénée ou un peracide organique le chromannol (I) où $R_3$ est S-$R_5$ selon un procédé connu pour former le chromannol (I) où $R_3$ est $SO_2R_5$;
- éventuellement on déshydrate les chromannols-3 ainsi obtenus en chromène correspondants par action d'un hydrure alcalin dans un solvant inerte, à une température comprise entre 50 et 100 °C;
- éventuellement on salifie le composé (I) selon un procédé connu lorsque $R_3$ contient un groupe amino.

**5.** Procédé de préparation des composés de formule (I) où $R_3$ représente un groupe $NHSO_2R_4$, caractérisé en ce que :

- on fait réagir l'époxyde (II) avec de l'ammoniac pour former le chromannol (I) où $R_3$ représente $-NH_2$;
- on substitue le groupe amine primaire selon un procédé connu par un chlorure d'acide sulfonique pour former le chromannol (I) où $R_3$ est un groupe $-NH-SO_2-R_4$;
- éventuellement on déshydrate les chromannols-3 ainsi obtenus en chromènes correspondants par action d'un hydrure alcalin dans un solvant inerte, à une température comprise entre 50 et 100 °C.

**6.** Procédé pour l'obtention des composés de formule (I), dans lesquels $R_1 + R_2$ représente une liaison, caractérisé en ce que :

- on soumet à une déshydratation les chromannols-3 correspondants
- et éventuellement on salifie le composé (I) selon un procédé connu lorsque $R_3$ contient un groupe amino.

**7.** Procédé de préparation des composés de formule (I) où

- Z représente le groupe cyano,
- $R_1 + R_2$ représente une liaison, et
- $R_3$ représente un groupe $NHR_4$ ou $SR_5$,

caractérisé en ce que :

- on traite le bromo-3 cyano-6 hydroxy-4 chromanne par une amine $NH_2R_4$ ou un thiol HS-$R_5$ au sein d'un solvant inerte et en présence d'un hydrure alcalin pour former directement le chromène correspondant (I) où $R_3$ est -NH-$R_4$ ou -$SR_5$, et éventuellement on salifie le composé (I) selon un procédé connu lorsque $R_3$ contient un groupe amino.

**8.** Composition pharmaceutique, caractérisée en ce qu'elle contient comme principe actif un dérivé du chromanne selon l'une quelconque des revendications 1 à 3 en combinaison avec un véhicule pharmaceutiquement acceptable.

**9.** Utilisation des dérivés selon l'une quelconque des revendications 1 à 3 pour la préparation de médicaments.

**10.** Utilisation des dérivés selon l'une quelconque des revendications 1 à 3 pour la préparation de médicaments utiles pour le traitement des états dépressifs.

**Claims**

**1.** Compounds of the formula :

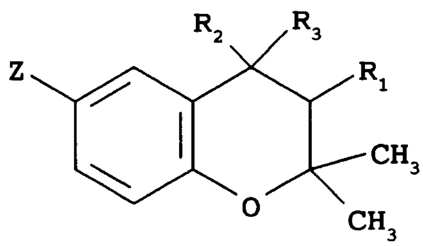

(I)

in which :

- Z represents a halogen or a cyano, acetyl, trifluoroacetyl, nitro, $C_1$-$C_4$ alkylthio, carboxyl, phosphono, dialkoxyphosphonyl, alkoxycarbonyl group, the alkylthio and alkoxy groups containing from 1 to 3 carbon atoms;
- $R_1$ denotes a hydroxyl group and $R_2$ represents hydrogen, or $R_1$ and $R_2$ taken together form an additional bond between the carbon atoms by which they are carried ; and
- $R_3$ represents
  . a group NH-X-$R_4$, in which X denotes a direct bond or an $SO_2$ group and $R_4$ represents a phenyl group which is unsubstituted or monosubstituted or disubstituted by a halogen, a nitro group, a $C_1$-$C_4$ alkyl group, a trifluoromethyl group, an amino group, a group -N(Alk)$_2$, a group - COOAlk, a group OAlk in which Alk denotes a $C_1$-$C_4$ alkyl group;
  . a group -S-$R_5$, in which $R_5$ represents a $C_1$-$C_4$ alkyl group, a $C_3$-$C_7$ cycloalkyl group, a phenyl group which is unsubstituted or monosubstituted or disubstituted by a halogen, a nitro group, a $C_1$-$C_4$ alkyl group, a trifluoromethyl group, an amino group, a group -N(Alk)$_2$, a group -COOAlk, a group OAlk, in which Alk denotes a $C_1$-$C_4$ alkyl group; or $R_5$ denotes a group -AlkN(Alk)$_2$ in which Alk is as defined above;
  . a group $SO_2R_5$, in which $R_5$ is as defined above ; as well as the salts which the compounds of formula (I) are capable of producing with the pharmaceutically acceptable acids when the substituent $R_3$ comprises an amino group.

**2.** Trans-4-(4-chloroanilino)-6-cyano-2,2-dimethyl-3-hydroxychroman.

**3.** 4-(4-Chlorophenylthio)-6-cyano-2,2-dimethyl-2H-chromene.

**4.** Method of preparing the compounds of formula (I) in which $R_3$ represents a group NH-$R_4$, $SR_5$ or $SO_2R_5$, characterized in that it comprises:

- reacting an amine $R_4NH_2$ or a thiol $HSR_5$ with the epoxide

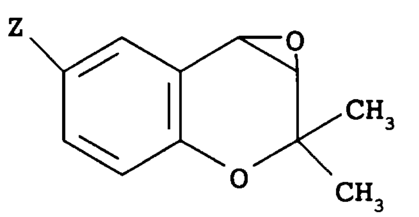

(II)

in an inert solvent, at a temperature of between 10 and 100° and in the presence of a basic

agent such as sodium hydride or benzyltrimethylammoniym hydroxide, to form the chromanol (I) in which $R_3$ represents a group NH-$R_4$ or $SR_5$;
- if desired, oxidizing the chromanol (I) in which $R_3$ is S-$R_5$ with hydrogen peroxide or an organic peracid, according to a known method, to form the chromanol (I) in which $R_3$ is $SO_2R_5$;
- if desired, dehydrating the resulting chroman-3-ols to give the corresponding chromenes by reaction with an alkali metal hydride in an inert solvent at a temperature of between 50 and 100 °C; and
- if desired, salifying the compound (I), according to a known method, when $R_3$ contains an amino group.

**5.** Method of preparing the compounds of formula (I) in which $R_3$ represents a group $NHSO_2R_4$, characterized in that it comprises:
- reacting the epoxide (II) with ammonia to form the chromanol (I) in which $R_3$ represents $-NH_2$;
- substituting the primary amine group, according to a known method, with a sulfonyl chloride to form the chromanol (I) in which $R_3$ is a group $-NH-SO_2-R_4$; and
- if desired, dehydrating the resulting chroman-3-ols to give the corresponding chromenes by reaction with an alkali metal hydride in an inert solvent at a temperature of between 50 and 100 °C.

**6.** Method of obtaining the compounds of formula I in which $R_1 + R_2$ represents a bond characterized in that it comprises:
- submitting to dehydratation the corresponding chroman-3-ols and
- if desired, salifying the compound (I), according to a known method, when $R_3$ contains an amino group.

**7.** Method of preparing the compounds of formula(I) in which :
- Z represents the cyano group ;
- $R_1 + R_2$ represents a bond and
- $R_3$ represents a group $NHR_4$ or $SR_5$,

characterized in that it comprises
- treating 3-bromo-6-cyano-4-hydroxychroman with an amine $NH_2R_4$ or a thiol HS-$R_5$, in an inert solvent and the presence of an alkali metal hydride, to form directly the corresponding chromene (I) in which $R_3$ is -NH-$R_4$ or -$SR_5$ and if desired, salifying the compound (I), according to a known method, when $R_3$ contains an amino group.

**8.** Pharmaceutical composition, characterized in that it contains as active principle, a chroman derivative according to any one of claims 1 to 3 in admixture with a pharmaceutically acceptable excipient.

**9.** Use of the derivatives according to anyone of claims 1 to 3 for the preparation of drugs.

**10.** Use of the derivatives according to any one of the claims 1 to 3 for the preparation of drugs useful for the treatment of depressive states.

**Patentansprüche**

**1.** Verbindungen der Formel

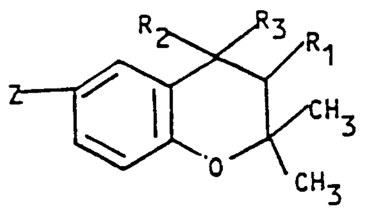

(I),

in der

- Z ein Halogen oder Cyano, Acetyl, Trifluoracetyl, Nitro, $C_{1-4}$-Alkylthio, Carboxy, Phosphono, Dialkoxyphosphonyl, Alkoxycarbonyl darstellt, wobei die Alkylthio- und Alkoxygruppen 1 bis 3 Kohlenstoffatome enthalten,
- $R_1$ eine Hydroxygruppe und $R_2$ Wasserstoff bedeutet oder $R_1$ und $R_2$ zusammen eine zusätzliche Bindung zwischen den Kohlenstoffatomen bilden, an die sie gebunden sind,
- $R_3$ darstellt:
  . eine Gruppe NH-X-$R_4$, in der X eine Einfachbindung oder eine Gruppe $SO_2$ bedeutet und $R_4$ eine Phenylgruppe darstellt, die ggf. einfach oder zweifach substituiert ist mit Halogen, Nitro, $C_{1-4}$-Alkyl, Trifluormethyl, Amino, - $N(Alk)_2$, -COOAlk, OAlk, worin Alk $C_{1-4}$-Alkyl bedeutet,
  . eine Gruppe -S-$R_5$, $R_5$ $C_{1-4}$-Alkyl, $C_{3-7}$-Cycloalkyl oder Phenyl darstellt, das ggf. einfach oder zweifach mit Halogen, Nitro, $C_{1-4}$-Alkyl, Trifluormethyl, Amino, -$N(Alk)_2$, -COOAlk oder OAlk, worin Alk $C_{1-4}$-Alkyl bedeutet, substituiert ist oder $R_5$ eine Gruppe -AlkN$(Alk)_2$ bedeutet, in der Alk wie oben definiert ist,
  . eine Gruppe $SO_2R_5$, worin $R_5$ wie oben definiert ist,

sowie ihre Salze mit pharmazeutisch akzeptablen Säuren, wenn der Substituent $R_3$ eine Aminogruppe enthält.

2. trans-4-(4-Chloranilino)-6-cyano-2,2-dimethyl-3-hydroxychroman.

3. 4-(4-Chlorphenylthio)-6-cyano-2,2-dimethyl-2H-chromen.

4. Verfahren zur Herstellung von Verbindungen der Formel (I), in der $R_3$ eine Gruppe NH-$R_4$, $SR_5$ oder $SO_2R_5$ darstellt, gekennzeichnet durch folgende Schritte:
   - Umsetzen des Epoxids

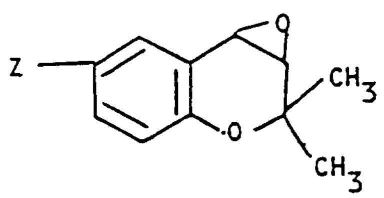

(II)

   mit einem bin $R_4NH_2$ oder einem Thiol $HSR_5$ in einem inerten Lösungsmittel bei einer Temperatur, die 10 bis 100 °C beträgt, und in Gegenwart eines basischen Mittels, wie z.B. Natriumhydrid oder Benzyltrimethylammoniumhydroxid, zur Erzeugung des Chromanols (I), worin $R_3$ NH-$R_4$ oder $SR_5$ darstellt,
   - gegebenenfalls Oxidieren des Chromanols (I), in dem $R_3$ S-$R_5$ ist, mit Wasserstoffperoxid oder einer organischen Peroxysäure zur Erzeugung des Chromanols (I), in dem $R_3$ $SO_2R_5$ ist,
   - gegebenenfalls Abspalten von Wasser aus den so hergestellten 3-Chromanolen unter Bildung der entsprechenden Chromene durch Einwirken eines Alkalihydrids in einem inerten Lösungsmittel bei einer Temperatur, die 50 bis 100 °C beträgt, und
   - gegebenenfalls Überfuhren der Verbindung (I) in ein Salz nach einem bekannten Verfahren, wenn $R_3$ eine Aminogruppe enthält.

5. Verfahren zur Herstellung von Verbindungen der Formel (I), in der $R_3$ eine Gruppe $NHSO_2R_4$ darstellt, gekennzeichnet durch folgende Schritte:
   - Umsetzen des Epoxids (II) mit Ammoniak zur Erzeugung von Chromanolen (I), worin $R_3$ -$NH_2$ darstellt,
   - Substitution der primären Aminogruppe nach einem bekannten Verfahren mit einem Sulfonsäurechlorid zur Erzeugung von Chromanolen (I), worin $R_3$ eine Gruppe -NH-$SO_2$-$R_4$ ist,
   - gegebenenfalls Abspalten von Wasser aus den so erhaltenen 3-Chromanolen unter Bildung der entsprechenden Chromene durch Einwirken eines Alkalihydrids in einem inerten Lösungsmittel bei einer Temperatur, die 50 bis 100 °C beträgt.

6. Verfahren zur Herstellung von Verbindungen der Formel (I), in der $R_1$ und $R_2$ eine Bindung darstellen, dadurch gekennzeichnet, daß die entsprechenden 3-Chromanole einer Wasserabspaltung unterzogen werden, und die Verbindung (I) gegebenenfalls nach einem bekannten Verfahren in ein Salz überge-

führt wird, wenn $R_3$ eine Aminogruppe enthält.

**7.** Verfahren zur Herstellung von Verbindungen der Formel (I), in der bedeuten:
- Z eine Cyanogruppe,
- $R_1$ und $R_2$ eine Bindung und
- $R_3$ eine Gruppe $NHR_4$ oder $SR_5$,

dadurch gekennzeichnet, daß
3-Brom-6-cyano-4-hydroxychroman mit einem Amin $NH_2R_4$ oder einem Thiol HS-$R_5$ in einem inerten Lösungsmittel und in Gegenwart eines Alkalihydrids behandelt wird zur unmittelbaren Erzeugung des entsprechenden Chromens (I), in dem $R_3$ -NH-$R_4$ oder -$SR_5$ darstellt, und die Verbindung (I) nach einem bekannten Verfahren in ein Salz übergeführt wird, wenn $R_3$ eine Aminogruppe enthält.

**8.** Pharmazeutische Zusammensetzungen, dadurch gekennzeichnet, daß sie als Wirkstoff ein Chromanderivat nach einem der Ansprüche 1 bis 3 in Kombination mit einem pharmazeutisch akzeptablen Vehikel enthält.

**9.** Verwendung der Derivate nach einem der Ansprüche 1 bis 3 zur Herstellung von Arzneimitteln.

**10.** Verwendung der Derivate nach einem der Ansprüche 1 bis 3 zur Herstellung von Arzneimitteln, die zur Behandlung von depressiven Zuständen verwendbar sind.